# EUROPEAN PATENT APPLICATION

(11) **EP 4 548 931 A1**
(43) Date of publication of application: **07.05.2025**
(21) Application number: 23830520.5
(22) Date of filing: 30.06.2023
(51) Int. Cl.: A61K 39/395, C07K 16/40, A61K 47/00

(54) **ANTI-PCSK9 ANTIBODY PREPARATION AND USE THEREOF**

(30) Priority: 30.06.2022 CN 202210768649
(71) Applicant: AD Pharmaceuticals Co., Ltd., Guangzhou, Guangdong 510555 (CN)
(72) Inventor: XIA, Yu, Zhongshan, Guangdong 528437 (CN); WANG, Zhao, Zhongshan, Guangdong 528437 (CN); XIA, Yu, Zhongshan, Guangdong 528437 (CN); LI, Baiyong, Zhongshan, Guangdong 528437 (CN)
(74) Representative: Altmann Stößel Dick Patentanwälte PartG mbB
(86) International application number: PCT/CN2023/105006
(87) International publication number: WO 2024/002357

(57) **Abstract**

The present invention relates to an anti-PCSK9 antibody preparation and use thereof in medicine. The preparation contains an anti-PCSK9 antibody and a buffer solution. The anti-PCSK9 antibody preparation can effectively inhibit the aggregation of the anti-PCSK9 antibody, thereby preventing the degradation of an antibody product and obtaining a stable injectable pharmaceutical preparation.

## Description

### TECHNICAL FIELD

The present disclosure relates to the field of biological pharmacy, and particularly, relates to an anti-PCSK9 antibody formulation and use thereof. Specifically, the present disclosure relates to a formulation of an anti-PCSK9 monoclonal antibody.

### BACKGROUND

PCSK9 (proproprotein convertase subtilisin kexin type 9) is a serine protease encoded by the PCSK9 gene, which is produced primarily by the liver. PCSK9 binds to LDL receptors (LDL-Rs) on the surface of hepatocytes and mediates the intracellular degradation of LDL-Rs, leading to an increase in plasma LDL-C levels. PCSK9 inhibitors (anti-PCSK9 antibodies) can interfere with the binding of PCSK9 to LDL-Rs, recover LDL-Rs to the liver surface, and promote the expression of more LDL-Rs in the liver, thereby lowering plasma LDL-C levels.

Anti-PCSK9 antibodies are used for the treatment of heterozygous familial hypercholesterolemia or clinical atherosclerotic cardiovascular disease and for patients in need of additional reduction of low-density lipoprotein cholesterol (LDL-C), and may also be used as adjuncts in the case of intolerance to statin therapy. Anti-PCSK9 antibodies may also improve cardiovascular conditions through other mechanisms, including reducing inflammation and oxidative stress within atherosclerotic plaques, and inhibiting prothrombotic pathways, which are particularly important for patients with acute coronary syndrome.

The clinical significance of inhibiting PCSK9 is that loss-of-function mutations in PCSK9 reduce LDL-C levels and significantly reduce the risk of cardiovascular events. In contrast, gain-of-function mutations in PCSK9 increase LDL-C levels and the risk of cardiovascular events. The reduction of LDL-C effects by HMG-CoA reductase mutations and PCSK9 mutations has a cumulative effect on reducing the rate of cardiovascular events, that is, the efficacy of anti-PCSK9 antibodies and statins may be additive.

A meta-analysis included 24 randomized trials (n = 10,159), covering a variety of clinical cases including: cases with familial hypercholesterolemia; cases with other types of hypercholesterolemia; statin-intolerant cases with hypercholesterolemia; and cases with intensive and non-intensive statin therapy and no statin therapy. The analysis found that anti-PCSK9 antibodies reduced all-cause mortality (OR 0.45, 95% CI 0.23-0.86), cardiovascular mortality (OR 0.50, CI 0.23-1.10), and myocardial infarction (OR 0.49, CI 0.26-0.93). The heterogeneity of the results of the included trials was not statistically significant, suggesting that anti-PCSK9 antibodies, like statins, have similar therapeutic effects for various clinical conditions and cardiovascular disease-based risks.

Only two PCSK9 inhibitors have been approved for marketing in the world, namely Amgen's evolocumab (trade name: Repatha) and alirocumab (trade name: Praluent) jointly developed by Sanofi and Regeneron, and both products are anti-PCSK9 monoclonal antibodies. There is currently no PCSK9 inhibitor as a small molecule chemical drug on the market. The prevalence of dyslipidemia among Chinese adults is greatly increased, and according to the "Report on Cardiovascular Diseases in China 2018", the prevalence of dyslipidemia among Chinese adults has reached 40.4% in 2012. There is still much room for improvement in the awareness rate, treatment rate and control rate of dyslipidemia among Chinese adults. Anti-PCSK9 antibodies can meet the clinical needs of people with dyslipidemia and poor control. Therefore, it is necessary to conduct in-depth research on this target antibody in order to obtain a more stable, more effective, and clinically convenient formulation.

### SUMMARY

A purpose of the present disclosure is to provide a stable anti-PCSK9 antibody formulation.

Specifically, the present disclosure relates to the following aspects:
1. An anti-PCSK9 antibody formulation, comprising an anti-PCSK9 antibody or an antigen-binding fragment thereof, at least one buffer, at least one stabilizer, and at least one surfactant, wherein the anti-PCSK9 antibody comprises HCDR1, HCDR2, and HCDR3 comprised by a heavy chain variable region set forth in SEQ ID NO: 8, and the antibody comprises LCDR1, LCDR2, and LCDR3 comprised by a light chain variable region set forth in SEQ ID NO: 10, preferably the anti-PCSK9 antibody comprises HCDR1 set forth in SEQ ID NO: 1, HCDR2 set forth in SEQ ID NO: 2, and HCDR3 set forth in SEQ ID NO: 3, and LCDR1 set forth in SEQ ID NO: 4, LCDR2 set forth in SEQ ID NO: 5, and LCDR3 set forth in SEQ ID NO: 6 according to an IMGT numbering system, wherein a sequence set forth in SEQ ID NO: 5 is GVI, and
   preferably the antigen-binding fragment is selected from Fab fragment, Fab' fragment, F(ab)' fragment, Fv fragment, isolated CDR region, single chain Fv molecule (scFv), F(ab')₂, Fd, dAb, Fab/c, bivalent antibody, and domain antibody.
2. The anti-PCSK9 antibody formulation according to item 1, wherein the buffer is selected from one or more of histidine buffer, citrate buffer, succinate buffer, acetate buffer, arginine buffer, and phosphate buffer, preferably the buffer is selected from 1-20 mM of a sodium citrate, and 1-20 mM of histidine and/or a histidine hydrochloride, preferably the histidine has a content of 1-7.4 mM, preferably 3-5.4 mM, more preferably 4-4.4 mM, and most preferably 4.2 mM, preferably the histidine hydrochloride has a content of 5-16.6 mM, preferably 8-13.6 mM, more preferably 10-11.6 mM, and most preferably 10.8 mM, and preferably the anti-PCSK9 antibody formulation has a pH of 5.0-6.0, preferably 5.2-5.8, and most preferably a pH of 5.5.
3. The anti-PCSK9 antibody formulation according to any one of items 1-2, wherein the stabilizer is selected from one or more of a saccharide, an amino acid, a polyol, an antioxidant, a preservative, a cyclodextrin, a polyethylene glycol (e.g., PEG3000, PEG3350, PEG4000, PEG6000), an albumin (e.g., human serum albumin (HSA), bovine serum albumin (BSA)), a salt (e.g., sodium chloride, calcium chloride), and an integrating agent (e.g., EDTA), preferably the stabilizer is selected from one or more of a saccharide, and the saccharide may be selected from sucrose and/or trehalose: preferably the sucrose has a content of 5-100 mg/mL, preferably 20-100 mg/mL or 40-80 mg/mL, more preferably 54-66 mg/mL, 55-65 mg/mL, or 57-63 mg/mL, and most preferably 60 mg/mL, the trehalose has a content of 5-100 mg/mL, preferably 5-55 mg/mL or 15-45 mg/mL, more preferably 25-35 mg/mL, 27-33 mg/mL, or 28.5-31.5 mg/mL, and most preferably 30 mg/mL, and preferably when the sucrose and the trehalose are used simultaneously, the sucrose and the trehalose have a total concentration of 85-95 mg/mL, preferably 90 mg/mL.
4. The anti-PCSK9 antibody formulation according to any one of items 1-3, wherein the surfactant is selected from a polyoxyethylene sorbitan fatty acid ester (Tween), e.g. polysorbate 20 and polysorbate 80, and preferably the polysorbate 80 has a content of 0.02%-0.08% (w/w), more preferably 0.025%-0.075% or 0.04%-0.06% (w/w), and most preferably 0.05% (w/w).
5. The anti-PCSK9 antibody formulation according to any one of items 1-4, wherein the anti-PCSK9 antibody formulation further comprises water for injection.
6. The anti-PCSK9 antibody formulation according to any one of items 1-5, wherein the anti-PCSK9 antibody or the antigen-binding fragment thereof has a concentration of 10-150 mg/mL, preferably 50-150 mg/mL or 80-120 mg/mL, more preferably 90-110 mg/mL, and most preferably 100 mg/mL.
7. The anti-PCSK9 antibody formulation according to any one of items 1-5, wherein the anti-PCSK9 antibody formulation comprises 100 mg/mL of the anti-PCSK9 antibody, 4.2 mM of the histidine, 10.8 mM of the histidine hydrochloride, 60 mg/mL of the sucrose, 30 mg/mL of the trehalose, and 0.05% (w/w) of the polysorbate 80, with the pH of 5.5.
8. The anti-PCSK9 antibody formulation according to any one of items 1-6, wherein the anti-PCSK9 antibody formulation further comprises a preservative, preferably the preservative is used in a quantity of about 0.001% to about 2% (w/v), preferably the preservative is selected from ethanol, benzyl alcohol, phenol, *m*-cresol, *p*-chloro-*m*-cresol, methyl paraben, propyl paraben, or benzalkonium chloride.
9. The anti-PCSK9 antibody formulation according to any one of items 1-6, wherein the anti-PCSK9 antibody formulation further comprises an antibacterial agent and an antifungal agent, e.g., chlorobutanol and sorbic acid.
10. The anti-PCSK9 antibody formulation according to any one of items 1-8, wherein the anti-PCSK9 antibody formulation is in a form suitable for injection (preferably subcutaneous injection).
11. A method for preparing an anti-PCSK9 antibody formulation, comprising mixing an anti-PCSK9 antibody or an antigen-binding fragment thereof with at least one buffer, at least one stabilizer, and at least one surfactant, and adjusting a pH of the formulation with an acid or a base to 5.0-6.0, preferably 5.2-5.8, and most preferably a pH of 5.5.
12. The method according to item 11, wherein the anti-PCSK9 antibody or the antigen-binding fragment thereof is as described in item 1 or 6, the buffer is as described in item 2, the stabilizer is as described in item 3, and the surfactant is as described in item 4.
13. The method according to item 11 or 12, wherein the surfactant is a non-ionic surfactant (e.g., polysorbate 80), and the non-ionic surfactant is added last and then a volume is determined.

In the present disclosure, the expression "drug formulation" also denotes an anti-PCSK9 antibody formulation.

In the drug formulation described herein, the anti-PCSK9 antibody or the antigen-binding fragment thereof comprises any one or more CDR region sequences selected from the following, or amino acid sequences having at least 85% (e.g., at least 86%, 87%, 88%, 89%, 90%, 91%, 92%, 93%, 94%, 95%, 96%, 97%, 98%, 99%) sequence homology thereto and having same activity:
In a specific embodiment, the anti-PCSK9 antibody comprises a heavy chain variable region and a light chain variable region, the antibody comprises HCDR1, HCDR2 and HCDR3 comprised in the heavy chain variable region set forth in SEQ ID NO: 8, and the anti-PCSK9 antibody comprises LCDR1, LCDR2 and LCDR3 comprised in the light chain variable region set forth in SEQ ID NO: 10, and preferably, the anti-PCSK9 antibody comprises HCDR1 set forth in SEQ ID NO: 1, HCDR2 set forth in SEQ ID NO: 2, and HCDR3 set forth in SEQ ID NO: 3, and LCDR1 set forth in SEQ ID NO: 4, LCDR2 set forth in SEQ ID NO: 5, and LCDR3 set forth in SEQ ID NO: 6.

The amino acid sequences are shown in Table 1 below:

**Table 1: CDR sequences comprised by anti-PCSK9 antibody**

| Domain | | Anti-PCSK9 antibody | |
|---|---|---|---|
| | | Sequence | SEQ ID NO. |
| VH | HCDR1 | GFTFSSYS | 1 |
| | HCDR2 | ISSSSSYI | 2 |
| | HCDR3 | AREYDFWSAYYDAFDV | 3 |
| VL | LCDR1 | SRNIGGGND | 4 |
| | LCDR2 | GVI | 5 |
| | LCDR3 | QSFDGSLSGSV | 6 |

The DNA sequence of the heavy chain variable region of the anti-PCSK9 antibody is as follows (369 bp):

GAGGTGCAGCTGGTGGAGTCTGGAGGAGGCCTGGTGCAGCCCGGAAGA
TCTCTGAGACTGAGTTGCGCCGCTTCAGGATTCACCTTTAGCTCCTACAG
CATGAACTGGGTGCGGCAGGCTCCTGGCAAGGGGCTGGAGTGGGTCTCC
GGAATCTCTAGTTCAAGCTCCTACATTAGCTATGCAGACTCCGTCCAGGG
AAGGTTCACCATCTCTCGCGATAACGGCAAGAACAGCCTGTATCTGCAG
ATGAACAGCCTGCGAGCAGAGGACACAGCCCTGTACTTCTGTGCCAGAG
AATATGACTTCTGGTCCGCCTATTACGACGCCTTCGATGTCTGGGGACAG
GGGACTATGGTCACTGTCTCAAGC (SEQ ID NO: 7)

The sequence of the heavy chain variable region encoded by the above DNA sequence is as follows (123 aa):

EVQLVESGGGLVQPGRSLRLSCAASGFTFSSYSMNWVRQAPGKGLEWVSG
ISSSSSYISYADSVQGRFTISRDNGKNSLYLQMNSLRAEDTALYFCAREYDF
WSAYYDAFDVWGQGTMVTVSS (SEQ ID NO: 8)

The DNA sequence of the light chain variable region of the anti-PCSK9 antibody is as follows (333 bp):

CAGAGCGAACTGACTCAGCCAAGAAGCGTCAGTGGATCACCTGGCCAG
AGCGTGACAATCTCCTGCACCGGCACAAGCAGGAACATTGGCGGGGGA
AATGACGTCCACTGGTACCAGCAGCATCCAGGGAAGGCCCCCAAACTGC
TGATCTCCGGAGTGATTGAGCGGAGCTCCGGCGTCCCCGATAGATTCAGC

GGGTCCAAGTCTGGAAACACAGCTTCTCTGACTATCAGTGGCCTGCAGG
CAGAGGACGAAGCCGATTACTATTGCCAGTCTTTCGACGGCAGTCTGTC
AGGGAGCGTGTTTGGCACTGGGACCGATGTGACCGTCCTG (SEQ ID NO:
9)

The sequence of the light chain variable region encoded by the above DNA sequence is as follows (111 aa):

QSELTQPRSVSGSPGQSVTISCTGTSRNIGGGNDVHWYQQHPGKAPKLLISG
VIERSSGVPDRFSGSKSGNTASLTISGLQAEDEADYYCQSFDGSLSGSVFGT
GTDVTVL (SEQ ID NO: 10)

The anti-PCSK9 antibody formulation described herein is a drug formulation suitable for subcutaneous injection.

In one embodiment of the present disclosure, the anti-PCSK9 antibody formulation comprises or consists of an anti-PCSK9 antibody, a buffer, a saccharide, and a surfactant, and optionally further comprises water for injection.

In one embodiment of the present disclosure, the components of the anti-PCSK9 antibody formulation are preferably as shown in Table 2 below:

**Table 2: components of anti-PCSK9 antibody formulation**

| Antibody | Concentration | Excipient 1 | Excipient 2 | Excipient 3 | Excipient 4 | Excipient 6 | pH value |
|---|---|---|---|---|---|---|---|
| PCSK9 | 100 mg/mL | 4.2 mM histidine | 10.8 mM histidine hydrochloride | 60 mg/mL sucrose | 30 mg/mL trehalose | 0.05% (w/w) polysorbate 80 | 5.5 |

The "antibody" described herein refers to an immunoglobulin, which is a tetrapeptide chain structure formed by two identical heavy chains and two identical light chains linked via interchain disulfide bonds.

The antibody of the present disclosure comprises a murine antibody, a chimeric antibody, and a humanized antibody, preferably a humanized antibody.

The term "antigen-binding fragment" of an antibody (or simply "antibody fragment") refers to one or more fragments of the antibody that retain the ability to specifically bind to an antigen (e.g., PCSK9). It has been shown that fragments of a full-length antibody may be used to perform the antigen-binding function of the antibody. An antigen-binding site may be produced by a recombinant DNA technique or by enzymatic or chemical cleavage of an intact immunoglobulin. In the present disclosure, examples of the antigen-binding fragment include a Fab fragment, a Fab' fragment, an F(ab)' fragment, an Fv fragment, an isolated CDR region, a single chain Fv molecule (scFv), F(ab')₂, Fd, dAb, Fab/c, a bivalent antibody, and a domain antibody.

The term "CDR" refers to one of the 6 hypervariable regions within the variable domain of an antibody which primarily contributes to antigen binding. One of the most common definitions of the 6 CDRs is provided by Kabat E. A. et al., (1991) Sequences of proteins of immunological interest. NIH Publication 91-3242. As used herein, the Kabat definition of the CDRs applies only to LCDR1, LCDR2, and LCDR3 of the light chain variable domain, and HCDR1, HCDR2, and HCDR3 of the heavy chain variable domain.

In the present disclosure, the antibody described herein may further comprise a light chain constant region, and the light chain constant region comprises a human or murine K, λ chain, or a variant thereof.

In the present disclosure, the antibody described herein may further comprise a heavy chain constant region, and the heavy chain constant region comprises human or murine IgG1, IgG2, IgG3, IgG4, or a variant thereof.

The term "chimeric antibody" refers to an antibody formed by fusing a variable region of a murine antibody with a constant region of a human antibody, which can reduce an immune response induced by the murine antibody. The chimeric antibody is established by first establishing a hybridoma secreting specific murine monoclonal antibody, then cloning a variable region gene from a murine hybridoma cell, then cloning a constant region gene of a human antibody as needed, ligating the murine variable region gene and the human constant region gene into a chimeric gene and then inserting into an expression vector, and finally expressing a chimeric antibody molecule in an eukaryotic system or a prokaryotic system.

The term "humanized antibody", also known as a CDR-grafted antibody, refers to an antibody produced by grafting a murine CDR sequence into a human antibody variable region framework, i.e., an antibody produced in framework sequences of different types of human germline antibodies. It can overcome a heterologous reaction induced by a chimeric antibody because of carrying a large number of murine protein components. Such framework sequences may be obtained from public DNA databases or published references that comprise germline antibody gene sequences.

The term "drug formulation" or "formulation" refers to such a product: it is in a form that allows biological activity of an active ingredient to be clearly effective, and it does not contain additional components that are toxic to a subject to which the formulation is to be administered.

The term "liquid" as used herein in conjunction with the formulation according to the present disclosure denotes a formulation that is liquid at atmospheric pressure at a temperature of at least about 20 °C to about 80 °C.

The term "stabilizer" denotes a pharmaceutically acceptable excipient that protects an active pharmaceutical ingredient and/or a formulation from chemical and/or physical degradation during manufacture, storage and use. The chemical and physical degradation pathways of protein drugs were reviewed by Cleland, J. L., M. F. Powell et al. (1993). "The development of stable protein formulations: a close look at protein aggregation, deamidation, and oxidation." Crit Rev Ther Drug Carrier Syst 10(4): 307-77, Wang, W. (1999). "Instability, stabilization, and formulation of liquid protein pharmaceuticals." Int J Pharm 185(2): 129-88, Wang, W. (2000). "Lyophilization and development of solid protein pharmaceuticals I J Pharm 203(1-2): 1-60, and Chi, E. Y, S. Kri shnan et al. (2003). "Physical stability of proteins in aqueous solution: mechanism and driving forces in nonnative protein aggregation." Pharm Res 20(9): 1325-36.

Preferably, the formulation of the present disclosure is a "stable" formulation, which represents such a formulation: a protein (e.g., antibody) contained therein substantially retains its physical and chemical stability, and thus its biological activity, after storage.

For example, no significant change is observed in the antibody formulation of the present disclosure at a refrigerated temperature of 2-8 °C for at least 12 months, particularly for 2 years, and more particularly for 3 years. For example, the criteria for stability are as follows: colorless to light yellow clear liquid in the result of a visual method; not less than 95% of a main peak in the detection result of size exclusion chromatography (SE-HPLC); not less than 95% of a sum of heavy chains and light chains in the detection result of reduced capillary electrophoresis (rCE-SDS); not less than 85% of a main peak in the detection result of non-reduced capillary electrophoresis (nrCE-SDS); and 50%-150% of a reference substance in the result of a biological activity assay (ELISA).

The terms "protein PCSK9" and "PCSK9" are used interchangeably and include variants, isoforms, species homologs, and analogs having at least one common epitope with PCSK9 of human PCSK9. The complete PCSK9 sequence may be found according to NCBI Reference Sequence: Genbank ID: NP_000567.1.

The terms "anti-PCSK9 antibody", "antibody against PCSK9", and "antibody against PCSK9 protein" refer to an antibody that is capable of binding to PCSK9 protein with sufficient affinity such that the antibody may be used as a diagnostic agent and/or a therapeutic agent in targeting PCSK9 protein. The term "binding to a PCSK9 protein" as used herein refers binding of an antibody to PCSK9 protein in a BIAcore assay (Pharmacia Biosensor AB, Uppsala, Sweden) or in an ELISA.

The term "surfactant" as used herein denotes a pharmaceutically acceptable excipient used to protect a protein formulation against physical stress (e.g., agitation and shear). Examples of the pharmaceutically acceptable surfactant comprise: a polyoxyethylene sorbitan fatty acid ester (Tween). Examples of the polyoxyethylene sorbitan-fatty acid ester are polysorbate 20 and polysorbate 80. The term "buffer" as used herein denotes a pharmaceutically acceptable excipient that stabilizes a pH of a drug formulation. Suitable buffers are well known in the art and may be found in literature. The preferred pharmaceutically acceptable buffer comprises, but is not limited to: a histidine buffer, a citrate buffer, a succinate buffer, an acetate buffer, an arginine buffer, a phosphate buffer, or a mixture thereof. The preferred buffer comprises a histidine/histidine hydrochloride buffer, which is adjusted for a pH with an acid or a base known in the art. The above buffer is generally used in a quantity of 1-100 mM, preferably 10-30 mM, and most preferably 20 mM. A pH of a drug formulation may be adjusted to a pH of 5.0-6.0, particularly to a pH of 5.2-5.8, and most particularly to a pH of 5.5, using an acid or a base known in the art (e.g., hydrochloric acid, acetic acid, phosphoric acid, sulfuric acid and citric acid, sodium hydroxide and potassium hydroxide), independently of the buffer used.

In certain embodiments, the drug formulation of the present disclosure may further comprise an antioxidant as a second stabilizer. An "antioxidant" is a pharmaceutically acceptable excipient that prevents oxidation of an active pharmaceutical ingredient. The antioxidant comprises, but is not limited to: integrins such as EDTA, citric acid, ascorbic acid, butylated hydroxytoluene (BHT), butylated hydroxyanisole (BHA), sodium sulfite, p-aminobenzoic acid, glutathione, propyl gallate, cysteine, methionine, ethanol, benzyl alcohol, and n-acetylcysteine.

The term "saccharide" as used herein denotes a monosaccharide or an oligosaccharide. The monosaccharide is a monomeric carbohydrate that may not be hydrolyzed by an acid, including the monosaccharide and a derivative thereof, such as an amino sugar. Examples of the monosaccharide comprise glucose, fructose, galactose, mannose, sorbose, ribose, deoxyribose, and neuraminic acid. The oligosaccharide is a branched or linear carbohydrate consisting of more than one monomeric sugar unit linked via a glycosidic bond. The monomeric sugar unit within the oligosaccharide may be the same or different. Depending on the number of the monomeric sugar unit, the oligosaccharide is a disaccharide, a trisaccharide, a tetrasaccharide, a pentasaccharide, and the like. Unlike a polysaccharide, the monosaccharide and the oligosaccharide are water-soluble. Examples of the oligosaccharide comprise sucrose, trehalose, lactose, maltose, and raffinose. Specifically, the saccharide is selected from sucrose and trehalose.

The term "amino acid" as used herein denotes a pharmaceutically acceptable organic molecule having an amino moiety located at an α-site of a carboxyl group. Examples of the amino acid comprise arginine, glycine, ornithine, lysine, histidine, glutamic acid, aspartic acid, isoleucine, leucine, alanine, phenylalanine, tyrosine, tryptophan, methionine, serine, and proline. The amino acid is generally used in the following quantity: a quantity of 5-200 mM, particularly a quantity of 5-100 mM, and more particularly a quantity of 5-70 mM.

The term "stabilizer" also comprises a cryoprotectant. The term "cryoprotectant" denotes a pharmaceutically acceptable excipient that protects an unstable active ingredient (e.g., protein) against a destabilizing condition during freeze-drying, subsequent storage, and reconstitution. The cryoprotectant comprises, but is not limited to, a saccharide, a polyol (e.g., sugar alcohol), and an amino acid. Specifically, the cryoprotectant may be selected from: the saccharide such as sucrose, trehalose, lactose, glucose, mannose, maltose, galactose, fructose, sorbose, raffinose, neuraminic acid, an amino sugar such as glucosamine, galactosamine, N-methyl glucosamine ("meglumine"), the polyol such as mannitol and sorbitol, and the amino acid such as arginine and glycine or a mixture thereof. The cryoprotectant is preferably a disaccharide, and specifically, is preferably sucrose and trehalose. The inventors have surprisingly found that the disaccharide is superior to the monosaccharide, the polyol, and the amino acid in stability for the formulation.

The drug formulation may also contain a tonicity agent. The term "tonicity agent" as used herein denotes a pharmaceutically acceptable tonicity agent for adjusting tonicity of a formulation. The formulation may be hypotonic, isotonic, or hypertonic, preferably isotonic. Isotonicity generally relates to the relative osmotic pressure of a solution, often relative to the osmotic pressure of human serum. An isotonic formulation is a liquid or a liquid reconstituted from a solid form (e.g., from a lyophilized form) and denotes a solution having the same tonicity as some other solution to which it is compared (such as a physiological saline solution and serum). A suitable tonicity agent comprises, but is not limited to, sodium chloride, potassium chloride, glycerin, and any component selected from the following: an amino acid, a saccharide, especially glucose. The tonicity agent is generally used in a quantity of about 5 mM to about 500 mM. Within the stabilizer and tonicity agent, there is a group of compounds that can act in two ways, that is, they may be both a stabilizer and a tonicity agent. Examples thereof can be found in the following set: a saccharide, an amino acid, a polyol, a cyclodextrin, a polyglycol, and a salt. An example of the saccharide that may be both a stabilizer and a tonicity agent is trehalose.

The drug formulation may also contain an adjuvant such as a preservative, a wetting agent, an emulsifying agent, and a dispersing agent. Prevention of the presence of microorganisms may be ensured by sterilization operations and by inclusion of different antibacterial and antifungal agents (e.g., methyl paraben, trichlorobutanol, phenol, sorbic acid, etc.). The preservative is generally used in an amount of about 0.001% to about 2% (w/v). The preservative comprises, but is not limited to: ethanol, benzyl alcohol, phenol, m-cresol, p-chloro-m-cresol, methyl paraben or propyl paraben, and benzalkonium chloride.

The anti-PCSK9 antibody drug formulation according to the present disclosure may be used for the treatment of heterozygous familial hypercholesterolemia or clinical atherosclerotic cardiovascular disease and for adults in need of additional reduction of low-density lipoprotein cholesterol (LDL-C), and may also be used as adjuncts in the case of intolerance to statin therapy. The anti-PCSK9 antibody may improve cardiovascular conditions through other mechanisms, including reducing inflammation and oxidative stress within atherosclerotic plaques, and inhibiting prothrombotic pathways.

The anti-PCSK9 antibody drug formulation according to the present disclosure may be administered intravenously (i.v.), subcutaneously (s.c.), or by any other parenteral administration means such as those known in the pharmaceutical art.

In view of high stability thereof, the drug formulation according to the present disclosure may be administered subcutaneously without the need for a filter (in-line filter).

The formulation for *in vivo* administration must be sterile. This may be effectively achieved by an aseptic manufacturing process that is not terminal sterilization.

The anti-PCSK9 antibody drug formulation according to the present disclosure may be prepared by methods known in the art, such as ultrafiltration-diafiltration, dialysis, addition and mixing, reconstitution, and a combination thereof. Preparation examples of the formulation according to the present disclosure may be found below.

### DETAILED DESCRIPTION

The embodiments of the present disclosure will be described in detail below with reference to the examples. Those skilled in the art will appreciate that the following examples are only for illustrating the present disclosure, and should not be construed as limitations to the scope of the present disclosure. Examples where the specific technologies or conditions are not specified are performed according to the technologies or conditions described in the publications of the art (e.g., see, Molecular Cloning: A Laboratory Manual, authored by J. Sambrook et al., and translated by Huang Peitang et al., third edition, Science Press) or according to the package insert. Reagents or instruments used are commercially available conventional products if the manufacturers thereof are not specified.

### Preparation Example 1. Preparation of Humanized Anti-PCSK9 Antibody

### 1. Design of antibodies

In order to prepare monoclonal antibody MAB1, the inventors designed a series of antibody sequences based on the known PCSK9 protein sequence (NP_777596.2) and three-dimensional crystal structure thereof. Through extensive screening and analyses, the antibody MAB1 that specifically binds to PCSK9 was finally obtained. The amino acid sequences of the heavy chain and the light chain variable regions of the monoclonal antibody and the encoding sequences thereof are set forth in SEQ ID NOs: 1-4 below.

The DNA sequence of the heavy chain VH of the designed MAB1 is as follows (369 bp):

GAGGTGCAGCTGGTGGAGTCTGGAGGAGGCCTGGTGCAGCCCGGAAGA
TCTCTGAGACTGAGTTGCGCCGCTTCAGGATTCACCTTTAGCTCCTACAG
CATGAACTGGGTGCGGCAGGCTCCTGGCAAGGGGCTGGAGTGGGTCTCC
GGAATCTCTAGTTCAAGCTCCTACATTAGCTATGCAGACTCCGTCCAGGG
AAGGTTCACCATCTCTCGCGATAACGGCAAGAACAGCCTGTATCTGCAG
ATGAACAGCCTGCGAGCAGAGGACACAGCCCTGTACTTCTGTGCCAGAG
AATATGACTTCTGGTCCGCCTATTACGACGCCTTCGATGTCTGGGGACAG
GGGACTATGGTCACTGTCTCAAGC (SEQ ID NO: 7)

The VH protein sequence encoded by the above DNA sequence is as follows (123 aa):
EVQLVESGGGLVQPGRSLRLSCAASGFTFSSYSMNWVRQAPGKGLEWVSG ISSSSSYISYADSVQGRFTISRDNGKNSLYLQMNSLRAEDTALYFCAREYDF WSAYYDAFDVWGQGTMVTVSS(SEQ ID NO: 8) The DNA sequence of the light chain VL of MAB1 is as follows (333 bp):

CAGAGCGAACTGACTCAGCCAAGAAGCGTCAGTGGATCACCTGGCCAG
AGCGTGACAATCTCCTGCACCGGCACAAGCAGGAACATTGGCGGGGGA
AATGACGTCCACTGGTACCAGCAGCATCCAGGGAAGGCCCCCAAACTGC
TGATCTCCGGAGTGATTGAGCGGAGCTCCGGCGTCCCCGATAGATTCAGC
GGGTCCAAGTCTGGAAACACAGCTTCTCTGACTATCAGTGGCCTGCAGG
CAGAGGACGAAGCCGATTACTATTGCCAGTCTTTCGACGGCAGTCTGTC
AGGGAGCGTGTTTGGCACTGGGACCGATGTGACCGTCCTG (SEQ ID NO:
9)

The VL protein sequence encoded by the above DNA sequence is as follows (111 aa):

QSELTQPRSVSGSPGQSVTISCTGTSRNIGGGNDVHWYQQHPGKAPKLLISG
VIERSSGVPDRFSGSKSGNTASLTISGLQAEDEADYYCQSFDGSLSGSVFGT
GTDVTVL (SEQ ID NO: 10)

The CDR sequences of the antibody are as follows:
HCDR1: GFTFSSYS (SEQ ID NO: 1);
HCDR2: ISSSSSYI (SEQ ID NO: 2);
HCDR3: AREYDFWSAYYDAFDV (SEQ ID NO: 3);
LCDR1: SRNIGGGND (SEQ ID NO: 4);
LCDR2: GVI (SEQ ID NO: 5); and
LCDR3: QSFDGSLSGSV (SEQ ID NO: 6).

### 2. Expression and purification of antibodies

The heavy chain cDNA sequence (the coding sequence of VH set forth in SEQ ID NO: 7; the constant region was Ig gamma-1 chain C region, ACCESSION: P01857) and the light chain cDNA sequence (the coding sequence of VL set forth in SEQ ID NO: 9; the constant region was Ig lambda-2 chain C regions; ACCESSION: P0CG05.1) of MAB1 were separately cloned into pUC57simple vectors (supplied by GenScript) to obtain pUC57simple-MAB1H and pUC57simple-MAB1L plasmids, respectively. pUC57simple-MAB1H and pUC57simple-MAB1L plasmids were digested (HindIII&EcoRI), and the heavy and light chains isolated by electrophoresis were separately subcloned into pcDNA3.1 vectors, and recombinant plasmids were extracted to co-transfect 293F cells. After 7 days of cell culture, the culture solution was centrifuged at high speed. The supernatant was concentrated and loaded onto a HiTrap MabSelect SuRe column. The protein was eluted in one step with an elution buffer. The target sample was isolated, and the buffer was exchanged into PBS to obtain the anti-PCSK9 antibody. In this example, the anti-PCSK9 antibody denotes the MAB1 antibody.

### Example 1: Preparation Process of Formulation

The composition of the anti-PCSK9 antibody formulation was as follows: the formulation consisted of a solute and a solvent, where the solute was the anti-PCSK9 antibody, histidine, a histidine hydrochloride, sucrose, trehalose, and polysorbate 80, and the solvent was water for injection; and the concentration of the anti-PCSK9 antibody in the formulation was 100 mg/mL, the concentration of the histidine in the formulation was 4.2 mM, the concentration of the histidine hydrochloride in the formulation was 10.8 mM, the concentration of the sucrose in the formulation was 60 mg/mL, the concentration of the trehalose in the formulation was 30 mg/mL, the concentration of the polysorbate 80 in the formulation was 0.05% (w/v), and the pH was 5.5.

When the anti-PCSK9 antibody formulation was formulated, the non-ionic surfactant polysorbate 80 in the solute was added at last and then a volume was determined, and the addition of other solutes was not in sequence. The anti-PCSK9 antibody formulation was aseptically subpackaged into a penicillin bottle, and capped with a rubber stopper and an aluminum-plastic cover, or the anti-PCSK9 antibody formulation was aseptically subpackaged into a liquid storage bag (Sartorius Flexboy) to obtain a finished product of the formulation.

### Example 2: Stability Experiment of Anti-PCSK9 Antibody Formulation

The stability experiment was performed on the anti-PCSK9 antibody formulation prepared in Example 1. The stability of the formulation under high temperature, light, freeze-thawing, agitation, and other conditions was investigated by size exclusion chromatography SEC-HPLC, capillary isoelectric focusing electrophoresis icIEF, reduced CE-SDS, non-reduced CE-SDS, activity and appearance observation of the formulation to characterize the chemical stability of the anti-PCSK9 antibody in the formulation under various environmental conditions separately, using the change in the percentage content of the main component and the main peak content of protein purity as determination means.

**Table 1. Chemical stability of anti-PCSK9 antibody in formulation under various environmental conditions**

| Detection item | High-temperature test (40±2 °C) detection time point | | | | |
|---|---|---|---|---|---|
| | Day 0 | Day 3 | Day 7 | Day 15 | Day 30 |
| Appearance | Colorless clear liquid | Colorless clear liquid | Colorless clear liquid | Colorless clear liquid | Colorless clear liquid |
| Protein content (mg/mL) | 103.6 | 96.0 | 100.8 | 97.1 | 98.2 |
| Purity (size exclusion chromatography) (%) | 97.9 | 96.8 | 96.1 | 94.4 | 92.3 |
| Purity (reduced CE-SDS) (%) | 98.4 | 98.4 | 98.4 | 98.4 | 97.0 |
| Purity (non-reduced CE-SDS) (%) | 95.3 | 93.5 | 92.1 | 94.8 | 93.1 |
| Purity (Charge heterogeneity) (%) | Acidic peak group: 13.7%; main peak group: 79.2%; basic peak group: 7.1% | Acidic peak group: 14.3%; main peak group: 74.0%; basic peak group: 11.8% | Acidic peak group: 16.1%; main peak group: 70.5%; basic peak group: 13.4% | Acidic peak group: 18.3%; main peak group: 62.6%; basic peak group: 19.1% | Acidic peak group: 22.3%; main peak group: 50.0%; basic peak group: 27.8% |
| Cytological activity (%) | 100.4 | - | - | - | 83.9 |

| | | | | | |
|---|---|---|---|---|---|
| Note: -: denoting no observation | | | | | |

**Table 2. Chemical stability of anti-PCSK9 antibody in formulation under various environmental conditions**

| Detection item | Freeze-thawing (-20 °C/25 °C) detection time point | | | |
|---|---|---|---|---|
| | Day 0 | A | B | C |
| Appearance | Colorless clear liquid | Colorless clear liquid | Colorless clear liquid | Colorless clear liquid |
| Protein content (mg/mL) | 103.6 | 99.6 | 106.5 | 97.4 |
| Purity (size exclusion chromatography) (%) | 97.9 | 98.0 | 98.2 | 97.9 |
| Purity (reduced CE-SDS) (%) | 98.4 | 98.3 | 98.4 | 98.4 |
| Purity (non-reduced CE-SDS) (%) | 95.3 | 93.0 | 93.0 | 92.8 |
| Purity (Charge heterogeneity) (%) | Acidic peak group: 13.7%; main peak group: 79.2%; basic peak group: 7.1% | Acidic peak group: 13.6%; main peak group: 77.8%; basic peak group: 8.6% | Acidic peak group: 13.6%; main peak group: 77.7%; basic peak group: 8.7% | Acidic peak group: 13.7%; main peak group: 77.7%; basic peak group: 8.6% |
| Cytological activity (%) | 104.8 | 91.3 | - | - |

| | | | | |
|---|---|---|---|---|
| Note: A: denoting 3 cycles of freeze-thawing; B: denoting 2 cycles of freeze-thawing; C: denoting 1 cycle of freeze-thawing; -: denoting no observation | | | | |

**Table 3. Chemical stability of anti-PCSK9 antibody in formulation under various environmental conditions**

| Detection item | Light (4500±500 Lux) detection time point | | |
|---|---|---|---|
| | Day 0 | Day 7 | Day 15 |
| Appearance | Colorless clear liquid | Light yellow clear liquid | Light yellow clear liquid |
| Protein content (mg/mL) | 103.6 | 104.1 | 97.3 |
| Purity (size exclusion chromatography) (%) | 97.9 | 95.8 | 94.4 |
| Purity (reduced CE-SDS) (%) | 98.4 | 98.1 | 97.7 |
| Purity (non-reduced CE-SDS) (%) | 95.3 | 91.9 | 89.7 |
| Purity (Charge heterogeneity) (%) | Acidic peak group: 13.7%; main peak group: 79.2%; basic peak group: 7.1% | Acidic peak group: 20.0%; main peak group: 68.2%; basic peak group: 11.8% | Acidic peak group: 40.8%; main peak group: 46.6%; basic peak group: 12.6% |
| Cytological activity (%) | 100.4 | - | 104.5 |

| | | | |
|---|---|---|---|
| Note: -: denoting no observation | | | |

**Table 4: Chemical stability of anti-PCSK9 antibody in formulation under various environmental conditions**

| Detection item | Agitation (300 rpm) detection time point | | |
|---|---|---|---|
| | Day 0 | 36 hours | 72 hours |
| Appearance | Colorless clear liquid | Colorless clear liquid | Colorless clear liquid |
| Protein content (mg/mL) | 103.6 | 97.8 | 97.0 |
| Purity (size exclusion chromatography) (%) | 97.9 | 98.2 | 98.2 |
| Purity (reduced CE-SDS) (%) | 98.4 | 98.4 | 98.4 |
| Purity (non-reduced CE-SDS) (%) | 95.3 | 94.0 | 94.0 |
| Purity (Charge heterogeneity) (%) | Acidic peak group: 13.7%; main peak group: 79.2%; basic peak group: 7.1% | Acidic peak group: 13.4%; main peak group: 78.1%; basic peak group: 8.6% | Acidic peak group: 13.7%; main peak group: 77.4%; basic peak group: 8.9% |
| Cytological activity (%) | 100.4 | - | 81.1 |

| | | | |
|---|---|---|---|
| Note: -: denoting no observation | | | |

The anti-PCSK9 antibody prepared in Preparation Example 1 was analyzed by size exclusion chromatography SEC-HPLC, capillary isoelectric focusing electrophoresis icIEF, reduced CE-SDS, non-reduced CE-SDS, and activity (ELISA) under the following experimental conditions: repeated freeze-thawing at -20 °C/25 °C for 3 cycles; agitating at 300 rpm for 3 days; and storing at 40 °C for 30 days; and the results of the change in the protein content, the change in the protein purity, and the change in the purity of the charge inhibitor when stored under 4500±500 Lux light for 15 days are shown in Tables 1-4.

According to the results in Table 1, the change in the protein content and the protein purity of the anti-PCSK9 antibody formulation decreased with the increase of storage time at a high temperature of 40 °C. In the SE-HPLC measurement on day 30, the main peak was 92.3%, and the antibody monomer was degraded by no more than 10%. In addition, through visual analysis, there was a light yellow clear liquid, which was consistent with that under normal conditions. The protein concentration of the formulation was 98.2 mg/mL, not more than +/-10% of the protein concentration under normal conditions. It was indicated that the formulation was still a stable liquid antibody formulation after being stored for 30 days at a high temperature of 40 °C.

According to the results in Table 2, the change of the protein content and the protein purity of the anti-PCSK9 antibody formulation did not change significantly after freeze-thawing. It was indicated that the formulation was still a stable liquid antibody formulation after three cycles of freeze-thawing.

According to the results in Table 3, the change in the protein content and the protein purity of the anti-PCSK9 antibody formulation decreased with the increase of storage time under light conditions. In the SE-HPLC measurement on day 15, the main peak was 94.4%, and the antibody monomer was degraded by no more than 10%. In addition, through visual analysis, there was a light yellow clear liquid, which was consistent with that under normal conditions. The protein concentration of the formulation was 97.3 mg/mL, not more than +/-10% of the protein concentration under normal conditions. It was indicated that the formulation was still a stable liquid antibody formulation after being stored for 15 days under light conditions.

According to the results in Table 4, the change in the protein content and the protein purity of the anti-PCSK9 antibody formulation did not change significantly after agitating treatment. It was indicated that the formulation was still a stable liquid antibody formulation after being agitated at 300 rpm for 3 days.

Summary: The results in Tables 1-4 show that the formulation has sufficient stability under the above storage conditions. It is shown that under various conditions that accelerated the chemical degradation and physical changes of the anti-PCSK9 antibody, the formulation formula can alleviate the decrease of chemical degradation rate of the contained anti-PCSK9 antibody and improve the physicochemical stability of the anti-PCSK9 antibody, so that the monoclonal antibody can stably exist in the formula of the formulation.

### Example 3. Comparison of Different Formulation Compositions and pH

(1) The purity of the formulations at different times was tested by SEC-HPLC using the different formula compositions shown in Table 5, respectively, and the results are shown in Table 6. AK102 denotes the anti-PCSK9 antibody-MAB1 antibody at a concentration of 100 mg/mL.

**Table 5. Compositions of anti-PCSK9 antibody mixed formula screening**

| Formula name | buffer | Trehalose | Sucrose | Tween 80 | PH |
|---|---|---|---|---|---|
| AK102-A | 15 mmol/L histidine | 10mg/mL | 80mg/mL | 0.05% | 5.5 |
| AK102-B | 15 mmol/L histidine | 30mg/mL | 60mg/mL | 0.05% | 5.5 |
| AK102-C | 15 mmol/L histidine | 50mg/mL | 40mg/mL | 0.05% | 5.5 |
| AK102-D | 15 mmol/L histidine | 70mg/mL | 20mg/mL | 0.05% | 5.5 |

**Table 6. SEC-HPLC purity results of anti-PCSK9 antibody formula screening**

| Main (%) | T0 | 40 °C3D | 40 °C1W | 40 °C2W |
|---|---|---|---|---|
| AK102-A | 98.4 | 98.3 | 98.0 | 98.5 |
| AK102-B | 98.4 | 98.1 | 97.9 | 98.2 |
| AK102-C | 98.5 | 98.2 | 97.9 | 98.0 |
| AK102-D | 98.4 | 98.1 | 97.8 | 97.6 |

As can be seen from Table 6, the formulations within the range of the component content of the present disclosure were all ensured good stability.

(2) Solution pH is one of the most important parameters affecting the physicochemical stability of biopharmaceuticals. The pH can regulate the charge distribution on the protein surface, thereby affecting the intramolecular and intermolecular forces of biopharmaceuticals, and at the same time affecting the rate of chemical modification of antibody drugs. It is one of the most critical properties of biopharmaceutical formulations.

According to the formulation formulas in Table 7, Tm tests and high-temperature degradation were performed to discover the stability differences of different formulas.

**Table 7. Different formula compositions of anti-PCSK9 antibody**

| Formula | Anti-PCSK9 antibody (mg/mL) | buffer | Stabilizer | Surfactant | pH |
|---|---|---|---|---|---|
| F1 | 14.7 | 20 mmol/L sodium citrate | 100 mmol/L sodium chloride | 0.05% polysorbate 80 | 6.0 |
| F2 | 13.9 | 20 mmol/L sodium citrate | 100 mmol/L sodium chloride | 0.05% polysorbate 80 | 5.5 |
| F3 | 14.8 | 20 mmol/L sodium citrate | 100 mmol/L sodium chloride | 0.05% polysorbate 80 | 5.2 |
| F4 | 14.7 | 20 mmol/L histidine | 100 mmol/L sodium chloride | 0.05% polysorbate 80 | 6.3 |
| F5 | 15.3 | 20 mmol/L histidine | 100 mmol/L sodium chloride | 0.05% polysorbate 80 | 6.0 |
| F6 | 15.4 | 20 mmol/L histidine | 100 mmol/L sodium chloride | 0.05% polysorbate 80 | 5.5 |
| F8 | 15.2 | 20 mmol/L histidine | 90 mg/mL sucrose | 0.05% polysorbate 80 | 5.5 |
| F9 | 13.9 | 20 mmol/L sodium citrate | 90 mg/mL sucrose | 0.05% polysorbate 80 | 5.5 |
| F10 | 13.8 | 20 mmol/L histidine | 150 mmol/L arginine hydrochloride | 0.05% polysorbate 80 | 5.5 |

### (2.1) Tm value tests

The protein melting temperature (Tm) reflects the thermal stability of biopharmaceuticals in aqueous solutions. The higher the Tm, the lower the possibility of conformational change under actual storage conditions. The Tm values of the anti-PCSK9 antibody in a buffer at different pH were investigated. The formulas in Table 8 were formulated by using 5 µL of the anti-PCSK9 antibody in the formulas in Table 7, and adding 5 µL of SYPRO Orange (20X) and 90 µL of a buffer, respectively. The Tm value of the sample was determined by a method similar to a differential scanning fluorimetry technique (DSF). The results are shown in Table 8.

**Table 8. Tm values of formulations at different pH conditions**

| **Formula** | **Tm1 (°C)** | **Tm2 (°C)** |
|---|---|---|
| F1 | 54.2 | 68.3 |
| F2 | 54.3 | 66.3 |
| F3 | 55.2 | 64.3 |
| F4 | 54.3 | 67.3 |
| F5 | 54.3 | 65.3 |
| F6 | 55.2 | 63.3 |
| F8 | 57.2 | 66.3 |
| F9 | 58.4 | 65.8 |
| F10 | 54.2 | 63.3 |

### (2.2) High-temperature acceleration studies of samples of different formulas

The formulations of different formulas in Table 7 were placed in a sample test chamber at 40±2 °C for accelerated damage for 3 days, and the stability of different formulations was investigated by appearance and SEC-HPLC.

The detection results are summarized in Table 9 and Table 10.

**Table 9. Changes in appearance of different formulations in high-temperature tests**

| Formula | T0* | 40C3d** |
|---|---|---|
| F1 | Clear solution | Turbid |
| F2 | Clear solution | Turbid |
| F3 | Clear solution | Turbid |
| F4 | Clear solution | Turbid |
| F5 | Clear solution | Turbid |
| F6 | Clear solution | Turbid |
| F8 | Clear solution | Clear solution |
| **F9** | Clear solution | Clear solution |
| F10 | Clear solution | Turbid |

| | | |
|---|---|---|
| Note: *T0 denotes the results at time 0; **40C3d denotes the results after 3 days of standing at 40 °C. | | |

**Table 10. Changes in SEC-HPLC purity in high-temperature tests of different**

| formulations | | | | | | |
|---|---|---|---|---|---|---|
| Formulation | T0* | | | 40C3d** | | |
| | Polymer (%) | Monomer (%) | Fragment (%) | Polymer (%) | Monomer (%) | Fragment (%) |
| F1 | 0 | 100 | 0 | 1.8 | 98.0 | 0.1 |
| F2 | 0 | 100 | 0 | 1.0 | 98.9 | 0.1 |
| F3 | 0 | 100 | 0 | 0.6 | 99.2 | 0.2 |
| F4 | 0 | 100 | 0 | 3.0 | 96.7 | 0.4 |
| F5 | 0 | 99.9 | 0.1 | 2.3 | 97.3 | 0.4 |
| F6 | 0 | 99.8 | 0.2 | 1.5 | 98.0 | 0.5 |
| F8 | 0 | 100 | 0 | 0.2 | 99. 6 | 0.2 |
| F10 | 0 | 100 | 0 | 79.9 | 20.1 | 0 |

| | | | | | | |
|---|---|---|---|---|---|---|
| Note: *T0 denotes the results at time 0; **40C3d denotes the results after 3 days of standing at 40 °C. | | | | | | |

According to the results of Tm measurement and the results of high-temperature acceleration study, only formula F8 still had clear appearance after acceleration, and other formulas were turbid, which were not suitable for the preservation of AK102. At the same time, the SEC-HPLC purity showed that the purity of formula F8 did not change significantly after 3 days of accelerated damage. It was preliminarily evaluated that AK102 had good stability under conditions of a polyhydroxyl protective agent and a pH of 5.5. According to this result, the screening and optimization of polyhydroxyl structural protective agent was performed.

### Screening and optimization of protective agents

According to the common protective agents of antibody drugs, the three formulas in Table 11 were designed, the samples of different formulas were placed at 40±2 °C for stability investigation, and the effects of different protective agents on the stability of AK102 were compared to select the optimal formula.

**Table 11. Compositions of AK102 formula screening**

| Formula | AK102(mg/mL) | buffer | Protective agent | Surfactant | pH |
|---|---|---|---|---|---|
| F11 | 15.2 | 15 mM histidine | 90 mg/mL sucrose | 0.05% polysorbate 80 | 5.5 |
| F2 | 8.3 | 15 mM histidine | 95 mg/mL trehalose | 0.05% polysorbate 80 | 5.5 |
| F13 | 8.5 | 15 mM histidine | 41 mg/mL sorbitol | 0.05% polysorbate 80 | 5.5 |

After the samples were damaged at 40±2 °C, the samples were taken separately at week 1, week 2, week 4, and week 8 for SEC-HPLC and CE-SDS analysis, and the changes of the three formulas were compared. The detection results are shown in Tables 12 and 13.

**Table 12. SEC-HPLC purity results of AK102 formula screening**

| Formula | T0 | Week 1 | Week 2 | Week 4 | Week 8 |
|---|---|---|---|---|---|
| F11 | 100 | 99.3 | 97.9 | 91.0 | (No detection) |
| F12 | 100 | 100 | 99.0 | 97.1 | 94.1 |
| F13 | 100 | 100 | 98.4 | 89.6 | 85.2 |

**Table 13. Reduced CE-SDS purity results of AK102 formula screening**

| Formula | T0 | Week 1 | Week 2 | Week 4 | Week 8 |
|---|---|---|---|---|---|
| F11 | 90.9 | 89.1 | 91.0 | 87.5 | (No detection) |
| F12 | 90.8 | 88.3 | 92.2 | 91.8 | 84.5 |
| F13 | 89.6 | 86.6 | 89.0 | 85.3 | 66.6 |

Summary: At 8 weeks of acceleration, no relevant detection analysis was performed on formula F11. From the SEC-HPLC and CE-SDS results, the stability of F12 (trehalose) was significantly better than that of formula F13 (sorbitol), and the results suggested that AK102 has better stability in trehalose formula.

From the perspective of process costs, the mixture of sucrose and trehalose was selected as the protective agent of AK102, and the mixing ratio was studied. The formulas of mixed saccharide protective agents with different proportions were designed as shown in Table 14. The concentration of AK102 samples in all the formulas was 100 mg/mL, which was the same as the final proposed packaging concentration.

**Table 14. Compositions of AK102 mixed formula screening**

| Formula name | buffer | Trehalose | Sucrose | Tween 80 | **PH** |
|---|---|---|---|---|---|
| AK102-A | 15 mmol/L histidine | 10mg/mL | 80mg/mL | 0.05% | 5.5 |
| AK102-B | 15 mmol/L histidine | 30mg/mL | 60mg/mL | 0.05% | 5.5 |
| AK102-C | 15 mmol/L histidine | 50mg/mL | 40mg/mL | 0.05% | 5.5 |
| AK102-D | 15 mmol/L histidine | 70mg/mL | 20mg/mL | 0.05% | 5.5 |

**Table 15. SEC-HPLC purity results of AK102 formula screening**

| Main (%) | T0 | 40 °C3D | 40 °C1W | 40 °C2W |
|---|---|---|---|---|
| AK102-A | 98.4 | 98.3 | 98.0 | 98.5 |
| AK102-B | 98.4 | 98.1 | 97.9 | 98.2 |
| AK102-C | 98.5 | 98.2 | 97.9 | 98.0 |
| AK102-D | 98.4 | 98.1 | 97.8 | 97.6 |

### Example 4. Stability Analysis of Different Formulation Formulas at Different Time Points

### Different formulations were formulated according to Table 16

**Table 16. Formulas of formulation samples F1-F19**

| Serial number | Protein content (mg/mL) | histidine (mM) | Trehalose (mg/mL) | Sucrose (mg/mL) | Polysorbate 80 (II) (%) |
|---|---|---|---|---|---|
| F1 | 100 | 18 | 27 | 66 | 0.025 |
| F2 | 100 | 15.75 | 27 | 54 | 0.075 |
| F3 | 100 | 18 | 33 | 54 | 0.075 |
| F4 | 90 | 18 | 33 | 66 | 0.025 |
| F5 | 90 | 13.5 | 33 | 54 | 0.075 |
| F6 | 90 | 18 | 27 | 60 | 0.075 |
| F7 | 110 | 13.5 | 27 | 60 | 0.075 |
| F8 | 110 | 15.75 | 27 | 66 | 0.025 |
| F9 | 90 | 18 | 30 | 54 | 0.025 |
| F10 | 100 | 13.5 | 33 | 66 | 0.05 |
| F11 | 90 | 13.5 | 27 | 60 | 0.025 |
| F12 | 110 | 18 | 27 | 54 | 0.025 |
| F13 | 90 | 15.75 | 30 | 66 | 0.075 |
| F14 | 110 | 15.75 | 33 | 60 | 0.025 |
| F15 | 110 | 13.5 | 30 | 54 | 0.025 |
| F16 | 110 | 18 | 30 | 66 | 0.075 |
| F17 | 100 | 15 | 30 | 60 | 0.05 |
| F18 | 100 | 15 | 30 | 60 | 0.05 |
| F19 | 100 | 15 | 30 | 60 | 0.05 |

| | | | | | |
|---|---|---|---|---|---|
| Note: F17-F19 are formulation numbers of the same formula for repeated experiments. | | | | | |

1 L of ultrafiltration buffer (without polysorbate 80 and antibody) for each of samples F1-19 was formulated according to the formulas in Table 16, and added with 1.9 mL of 6 M hydrochloric acid. The protein solutions in Table 16 were prepared using the ultrafiltration concentration system, and added with the 10% polysorbate 80 (II) solution in the corresponding sample in Table 16. About 90 mL of each formulation was prepared.

Filtration was performed using a 0.22 µm filter membrane. 30 samples (1.5 mL/sample) of each formula were prepared. 10 samples of each formula were sent for detection at point T0 (T0 denotes the time point for direct testing after formulation), and 20 samples were placed in a stability test chamber at 25 °C for acceleration tests (25°C3M denotes 3 months of standing at 25 °C). See Table 17 for detection items. The detection results are shown in Table 18-1, 18-2 (T0 detection results), and Tables 18-3 and 18-4 (25°C3M detection results).

Detection time points and items of sample formulations F1-F19:

| **Detection item** | **Method description** | **T0** | **25 °C3M** |
|---|---|---|---|
| Appearance | Visual method | X | X |
| pH value | pH value determination | X | X |
| SE-HPLC | Chromatography | X | X |
| icIEF | Imaged capillary isoelectric focusing electrophoresis | X | X |

**Table 18-1**

| Detection time point | Detection item | | Acceptance criteria | | | | | | | | | | |
|---|---|---|---|---|---|---|---|---|---|---|---|---|---|
| | | | | F1 | F2 | F3 | F4 | F5 | F6 | F7 | F8 | F9 | F10 |
| T0 | Appearance | | Colorless or light yellow clear liquid | Light yellow clear liquid with slight opalescence | Light yellow clear liquid with slight opalescence | Light yellow clear liquid with slight opalescence | Light yellow clear liquid with slight opalescence | Light yellow clear liquid with slight opalescence | Light yellow clear liquid with slight opalescence | Light yellow clear liquid with slight opalescence | Light yellow clear liquid with slight opalescence | Light yellow clear liquid with slight opalescence | Light yellow clear liquid with slight opalescence |
| | pH value | | 5.2 ~ 5.8 | 6.0 | 5.7 | 5.9 | 5.7 | 5.4 | 5.9 | 5.4 | 5.7 | 5.9 | 5.4 |
| | Purity (size exclusion chromatography) (%) | | Proportion of the main peak not less than 95.0% | 97.8 | 97.8 | 97.8 | 97.9 | 97.8 | 97.8 | 97.9 | 97.9 | 97.9 | 97.9 |
| | | | Polymer peak | 1.8 | 1.7 | 1.7 | 1.7 | 1.7 | 1.7 | 1.7 | 1.7 | 1.7 | 1.7 |
| | Charge heterogeneity | Report proportions of the main peak, acidic peak and basic peak | Acidic peak (%) | 20.8 | 19.4 | 20.5 | 19.8 | 19.7 | 20.2 | 20.1 | 20.1 | 20.2 | 20.1 |
| | | | Main peak (%) | 70.6 | 71.6 | 69.9 | 70.7 | 71.3 | 71.3 | 70.6 | 71.1 | 70.6 | 70.7 |
| | | | Basic peak (%) | 8.6 | 9.0 | 9.6 | 9.5 | 9.0 | 8.6 | 9.4 | 8.8 | 9.1 | 9.3 |
| | Protein content | | Should be 90.0-110.0 mg/mL | 99.5 | 100.3 | 101.1 | 87.7 | 89.9 | 89.9 | 108.8 | 108.1 | 97.9 | 99.0 |

**Table 18-2**

| Detection time point | Detection item | | Acceptance criteria | | | | | | | | | |
|---|---|---|---|---|---|---|---|---|---|---|---|---|
| | | | | F11 | F12 | F13 | F14 | F15 | F16 | F17 | F18 | F19 |
| T0 | Appearance | | Colorless or light yellow clear liquid | Light yellow clear liquid with slight opalescence | Light yellow clear liquid with slight opalescence | Light yellow clear liquid with slight opalescence | Light yellow clear liquid with slight opalescence | Light yellow clear liquid with slight opalescence | Light yellow clear liquid with slight opalescence | Light yellow clear liquid with slight opalescence | Light yellow clear liquid with slight opalescence | Light yellow clear liquid with slight opalescence |
| | pH value | | 5.2 ~ 5.8 | 5.4 | 5.9 | 5.7 | 5.7 | 5.4 | 5.8 | 5.6 | 5.6 | 5.6 |
| | Purity (size exclusion chromatography) (%) | | Proportion of the main peak not less than 95.0% | 98.1 | 97.9 | 98.1 | 97.9 | 98.0 | 97.9 | 98.0 | 98.0 | 98.0 |
| | | | Polymer peak | 1.6 | 1.7 | 1.6 | 1.7 | 1.7 | 1.7 | 1.7 | 1.7 | 1.7 |
| | Charge heterogeneity | Report proportions of the main peak, acidic peak and basic peak | Acidic peak (%) | 20.2 | 20.1 | 19.5 | 19.9 | 20.9 | 17.5 | 20.1 | 20.5 | 20.8 |
| | | | Main peak (%) | 70.8 | 71.3 | 71.5 | 70.7 | 70.1 | 73.4 | 70.8 | 70.7 | 70.2 |
| | | | Basic peak (%) | 9.0 | 8.6 | 9.0 | 9.5 | 9.0 | 9.2 | 9.1 | 8.8 | 9.0 |
| | Protein content | | Should be 90.0-110.0 mg/mL | 89.7 | 110.3 | 86.9 | 109.2 | 108.0 | 105.9 | 107.8 | 108.4 | 107.5 |

**Table 18-3**

| Detection time point | Detection item | | Acceptance criteria | | | | | | | | | | |
|---|---|---|---|---|---|---|---|---|---|---|---|---|---|
| | | | | F1 | F2 | F3 | F4 | F5 | F6 | F7 | F8 | F9 | F10 |
| 25 °C3M | Appearance | | Colorless or light yellow clear liquid | Light yellow clear liquid with slight opalescence | Light yellow clear liquid with slight opalescence | Light yellow clear liquid with slight opalescence | Light yellow clear liquid with slight opalescence | Light yellow clear liquid with slight opalescence | Light yellow clear liquid with slight opalescence | Light yellow clear liquid with slight opalescence | Light yellow clear liquid with slight opalescence | Light yellow clear liquid with slight opalescence | Light yellow clear liquid with slight opalescence |
| | pH value | | 5.2 ~ 5.8 | 5.9 | 5.7 | 5.9 | 5.7 | 5.4 | 5.8 | 5.4 | 5.7 | 5.8 | 5.4 |
| | Purity (size exclusion chromatography) (%) | | Proportion of the main peak not less than 95.0% | 97.3 | 96.9 | 97.0 | 97.3 | 97.0 | 97.2 | 96.9 | 97.2 | 97.3 | 97.1 |
| | | | Polymer peak | 2.1 | 2.5 | 2.4 | 2.1 | 2.4 | 2.2 | 2.5 | 2.3 | 2.1 | 2.2 |
| | Charge heterogeneity | Report proportions of the main peak, acidic peak and basic peak | Acidic peak (%) | 26.7 | 26.3 | 26.5 | 25.6 | 25.1 | 26.0 | 25.6 | 25.3 | 25.9 | 25.4 |
| | | | Main peak (%) | 54.2 | 52.9 | 53.2 | 53.9 | 52.8 | 54.1 | 52.7 | 54.3 | 54.6 | 53.0 |
| | | | Basic peak (%) | 19.1 | 20.8 | 20.3 | 20.4 | 22.1 | 19.9 | 21.6 | 20.4 | 19.5 | 21.6 |
| | Protein content | | Should be 90.0-110.0 mg/mL | 101.7 | 100.8 | 101.3 | 88.0 | 89.8 | 89.9 | 111.9 | 109.5 | 91.8 | 97.9 |

**Table 18-4**

| Detection time point | Detection item | | Acceptance criteria | | | | | | | | | |
|---|---|---|---|---|---|---|---|---|---|---|---|---|
| | | | | F11 | F12 | F13 | F14 | F15 | F16 | F17 | F18 | F19 |
| 25 °C3M | Appearance | | Colorless or light yellow clear liquid | Light yellow clear liquid with slight opalescence | Light yellow clear liquid with slight opalescence | Light yellow clear liquid with slight opalescence | Light yellow clear liquid with slight opalescence | Light yellow clear liquid with slight opalescence | Light yellow clear liquid with slight opalescence | Light yellow clear liquid with slight opalescence | Light yellow clear liquid with slight opalescence | Light yellow clear liquid with slight opalescence |
| | pH value | | 5.2 ~ 5.8 | 5.3 | 5.8 | 5.6 | 5.7 | 5.4 | 5.7 | 5.6 | 5.6 | 5.6 |
| | Purity (size exclusion chromatography) (%) | | Proportion of the main peak not less than 95.0% | 97.5 | 97.1 | 97.2 | 97.2 | 97.1 | 97.0 | 97.0 | 97.0 | 97.0 |
| | | | Polymer peak | 1.9 | 2.4 | 2.2 | 2.2 | 2.2 | 2.4 | 2.4 | 2.4 | 2.4 |
| | Charge heterogeneity | Report proportions of the main peak, acidic peak and basic peak | Acidic peak (%) | 24.9 | 25.8 | 26.0 | 26.6 | 25.4 | 26.9 | 26.2 | 26.5 | 26.4 |
| | | | Main peak (%) | 53.5 | 55 | 53.9 | 53.7 | 53.2 | 53.5 | 53.3 | 52.6 | 53.1 |
| | | | Basic peak (%) | 21.6 | 19.2 | 20.0 | 19.6 | 21.4 | 19.6 | 20.5 | 20.9 | 20.5 |
| | Protein content | | Should be 90.0-110.0 mg/mL | 90.1 | 111.1 | 87.6 | 109.3 | 109.5 | 110.1 | 110.4 | 109.4 | 109.8 |

According to the results in Table 18, the protein purity of the anti-PCSK9 antibody formulations within the range of the formulation formulas in Table 16 decreased with the increase of storage time at a high temperature of 25 °C. In SE-HPLC detection at month 3, the main peaks were all higher than 96%, and the antibody monomer was degraded by no more than 10%. In addition, through visual analysis, there was a light yellow clear liquid in appearance, which was consistent with that under normal conditions. It was indicated that the formulation was still a stable liquid antibody formulation after being stored for 30 days at 25 °C.

## Claims

1. An anti-PCSK9 antibody formulation, comprising an anti-PCSK9 antibody or an antigen-binding fragment thereof, at least one buffer, at least one stabilizer, and at least one surfactant, wherein the anti-PCSK9 antibody comprises HCDR1, HCDR2, and HCDR3 comprised by a heavy chain variable region set forth in SEQ ID NO: 8, and the antibody comprises LCDR1, LCDR2, and LCDR3 comprised by a light chain variable region set forth in SEQ ID NO: 10, preferably the anti-PCSK9 antibody comprises HCDR1 set forth in SEQ ID NO: 1, HCDR2 set forth in SEQ ID NO: 2, and HCDR3 set forth in SEQ ID NO: 3, and LCDR1 set forth in SEQ ID NO: 4, LCDR2 set forth in SEQ ID NO: 5, and LCDR3 set forth in SEQ ID NO: 6 according to an IMGT numbering system, wherein a sequence set forth in SEQ ID NO: 5 is GVI, and
preferably the antigen-binding fragment is selected from Fab fragment, Fab' fragment, F(ab)' fragment, Fv fragment, isolated CDR region, single chain Fv molecule (scFv), F(ab')₂, Fd, dAb, Fab/c, bivalent antibody, and domain antibody.

2. The anti-PCSK9 antibody formulation according to claim **1,** wherein the buffer is selected from one or more of histidine buffer, citrate buffer, succinate buffer, acetate buffer, arginine buffer, and phosphate buffer, preferably the buffer is selected from 1-20 mM of a sodium citrate, and 1-20 mM of histidine and/or a histidine hydrochloride, preferably the histidine has a content of 1-7.4 mM, preferably 3-5.4 mM, more preferably 4-4.4 mM, and most preferably 4.2 mM, preferably the histidine hydrochloride has a content of 5-16.6 mM, preferably 8-13.6 mM, more preferably 10-11.6 mM, and most preferably 10.8 mM, and preferably the anti-PCSK9 antibody formulation has a pH of 5.0-6.0, preferably 5.2-5.8, and most preferably a pH of 5.5.

3. The anti-PCSK9 antibody formulation according to any one of claims 1-2, wherein the stabilizer is selected from one or more of a saccharide, an amino acid, a polyol, an antioxidant, a preservative, a cyclodextrin, a polyethylene glycol (e.g., PEG 3000, PEG3350, PEG4000, PEG6000), an albumin (e.g., human serum albumin (HSA), bovine serum albumin (BSA)), a salt (e.g., sodium chloride, calcium chloride), and an integrating agent (e.g., EDTA), preferably the stabilizer is selected from one or more of a saccharide, and the saccharide may be selected from sucrose and/or trehalose: preferably the sucrose has a content of 5-100 mg/mL, preferably 20-100 mg/mL or 40-80 mg/mL, more preferably 54-66 mg/mL, 55-65 mg/mL, or 57-63 mg/mL, and most preferably 60 mg/mL, the trehalose has a content of 5-100 mg/mL, preferably 5-55 mg/mL or 15-45 mg/mL, more preferably 25-35 mg/mL, 27-33 mg/mL, or 28.5-31.5 mg/mL, and most preferably 30 mg/mL, and preferably when the sucrose and the trehalose are used simultaneously, the sucrose and the trehalose have a total concentration of 85-95 mg/mL, preferably 90 mg/mL.

4. The anti-PCSK9 antibody formulation according to any one of claims 1-3, wherein the surfactant is selected from a polyoxyethylene sorbitan fatty acid ester (Tween), e.g. polysorbate 20 and polysorbate 80, and preferably the polysorbate 80 has a content of 0.02%-0.08% (w/w), more preferably 0.025%-0.075% or 0.04%-0.06% (w/w), and most preferably 0.05% (w/w).

5. The anti-PCSK9 antibody formulation according to any one of claims 1-4, wherein the anti-PCSK9 antibody formulation further comprises water for injection.

6. The anti-PCSK9 antibody formulation according to any one of claims 1-5, wherein the anti-PCSK9 antibody or the antigen-binding fragment thereof has a concentration of 10-150 mg/mL, preferably 50-150 mg/mL or 80-120 mg/mL, more preferably 90-110 mg/mL, and most preferably 100 mg/mL.

7. The anti-PCSK9 antibody formulation according to any one of claims 1-5, wherein the anti-PCSK9 antibody formulation comprises 100 mg/mL of the anti-PCSK9 antibody, 4.2 mM of the histidine, 10.8 mM of the histidine hydrochloride, 60 mg/mL of the sucrose, 30 mg/mL of the trehalose, and 0.05% (w/w) of the polysorbate 80, with the pH of 5.5.

8. The anti-PCSK9 antibody formulation according to any one of claims 1-6, wherein the anti-PCSK9 antibody formulation further comprises a preservative, preferably the preservative is used in a quantity of about 0.001% to about 2% (w/v), preferably the preservative is selected from ethanol, benzyl alcohol, phenol, m-cresol, p-chloro-m-cresol, methyl paraben, propyl paraben, or benzalkonium chloride.

9. The anti-PCSK9 antibody formulation according to any one of claims 1-6, wherein the anti-PCSK9 antibody formulation further comprises an antibacterial agent and an antifungal agent, e.g., chlorobutanol and sorbic acid.

10. The anti-PCSK9 antibody formulation according to any one of claims 1-8, wherein the anti-PCSK9 antibody formulation is in a form suitable for injection (preferably subcutaneous injection).

11. A method for preparing an anti-PCSK9 antibody formulation, comprising mixing an anti-PCSK9 antibody or an antigen-binding fragment thereof with at least one buffer, at least one stabilizer, and at least one surfactant, and adjusting a pH of the formulation with an acid or a base to 5.0-6.0, preferably 5.2-5.8, and most preferably a pH of 5.5.

12. The method according to claim 11, wherein the anti-PCSK9 antibody or the antigen-binding fragment thereof is as described in claim 1 or 6, the buffer is as described in claim 2, the stabilizer is as described in claim 3, and the surfactant is as described in claim 4.

13. The method according to claim 11 or 12, wherein the surfactant is a non-ionic surfactant (e.g., polysorbate 80), and the non-ionic surfactant is added last and then a volume is determined.
